# FASCICULE DE BREVET EUROPEEN

(11) **EP 4 182 016 B1**
(45) Date de publication et mention de la délivrance du brevet: **04.06.2025**
(21) Numéro de dépôt: 21755526.7
(22) Date de dépôt: 17.07.2021
(51) Int. Cl.: A61N 5/06

(54) **DISPOSITIF DE PHOTOTHÉRAPIE MAINS LIBRES**
FREIHÄNDIGE LICHTTHERAPIEVORRICHTUNG
HANDS-FREE PHOTOTHERAPY DEVICE

(30) Priorité: 20.07.2020 FR 2007633
(43) Date de publication de la demande: 24.05.2023
(73) Titulaire: Lucibel SA, 76360 Barentin (FR); Lapidus, Olivier, 75008 Paris (FR)
(72) Inventeur: LAPIDUS, Olivier, 75008 PARIS (FR)
(74) Mandataire: Martin, Marie-Aude
(86) Numéro de dépôt international: PCT/FR2021/051343
(87) Numéro de publication internationale: WO 2022/018369

(56) Documents cités:
- EP-A2- 2 477 697
- EP-B1- 2 477 697
- WO-A1-2004/026400
- US-A1- 2011 251 658
- US-A1- 2015 375 007
- US-A1- 2016 310 757
- US-A1- 2017 216 577
- US-A1- 2018 021 592
- US-A1- 2019 262 628

## Description

La présente invention concerne un dispositif de traitement par photothérapie pour une région externe du corps d'un être vivant. Plus particulièrement mais non exclusivement, elle s'applique au traitement par photothérapie de la surface de tête d'un utilisateur, par exemple au traitement du cuir chevelu en tant que traitement de la calvitie.

De façon connue en soi, le traitement consiste en l'application d'un rayonnement lumineux présentant un spectre d'émission prédéfini directement sur le cuir chevelu. Ce traitement par photothérapie peut être appliqué également en combinaison avec un produit photo-sensibilisant appliqué sur la région à traiter quelques heures avant son exposition au rayonnement lumineux pour augmenter l'efficacité du traitement. On parle alors dans ce cas de photothérapie dynamique (connue également sous l'acronyme anglais PDT « Photo Dynamic The-rapy »).

Le rayonnement est de préférence sensiblement monochromatique ou présente un spectre d'émission compris dans une bande relativement étroite de longueurs d'onde. Généralement, le spectre d'émission comprend une longueur d'onde maximale située dans la lumière visible rouge ou encore dans la lumière visible bleue, ces longueurs d'onde ayant démontré une efficacité particulière pour le traitement de certaines affections de la peau.

Il a été ainsi observé au cours de nombreuses études scientifiques, qu'un traitement par photothérapie dans une plage de longueurs d'onde prédéfinie, notamment dans la lumière rouge ou bleue, permet d'agir de façon bénéfique sur de nombreux problèmes cutanés, tels que les cicatrices, les vergetures, l'acné, les rides et ridules.

Plus particulièrement, lors de l'application du rayonnement sur la peau, notamment dans la lumière rouge et plus particulièrement autour de la longueur d'onde 630 nm, les cellules de la région irradiées vont réagir selon un principe similaire à celui de la photosynthèse des végétaux. La lumière va stimuler les cellules, et plus spécifiquement certaines structures cellulaires de la cellule, telles que les mitochondries.

De façon connue en soi, les mitochondries jouent un rôle particulièrement important dans le processus de respiration cellulaire ainsi que dans le processus de transformation des nutriments en un vecteur énergétique désigné couramment par ATP (acronyme pour Adénosine Tri Phosphate). Dès lors, la stimulation des mitochondries par la lumière rouge contribue à une régénération des cellules grâce à l'amélioration du métabolisme cellulaire. Une telle régénération cellulaire va se manifester par exemple pour les cellules de la peau du visage par une augmentation de la production de collagène et d'élastine et par conséquent une diminution observable des rides et ridules.

Plus particulièrement, il a été observé que l'exposition du cuir chevelu à la lumière rouge, par exemple à la longueur d'onde de 630 nm, permet une stimulation de la repousse capillaire, une amélioration de la qualité et de la densité des cheveux ainsi qu'une stabilisation de la perte de cheveux.

On connaît déjà dans l'état de la technique, en particulier de la demande de brevet EP24477697 un équipement de traitement par photothérapie du cuir chevelu d'un utilisateur, comprenant un casque délimitant une extrémité ouverte par laquelle, en position d'utilisation, la tête de l'utilisateur est au moins partiellement engagée. WO 2004/026400 A1 divulgue un dispositif de photothérapie portatif mains libres, du type comprenant une unité principale de tête comportant un bandeau en forme d'arceau configuré pour être positionné au-dessus de la tête d'un utilisateur, dans lequel l'accrochage du bandeau aux unités d'oreille autorise une rotation relative du bandeau par rapport aux unités d'oreilles.

L'inconvénient d'un tel équipement est qu'il est particulièrement encombrant et peu ergonomique de sorte qu'il ne peut être porté en toute discrétion ou être aisément transporté en déplacement avec l'utilisateur.

L'invention a notamment pour but de remédier à ces inconvénients en proposant un dispositif de photothérapie ergonomique, esthétique et aisément transportable.

A cet effet, l'invention a pour un dispositif de photothérapie portatif mains libres, , du type comprenant une unité principale de tête comportant un bandeau en forme d'arceau configuré pour être positionné au-dessus de la tête d'un utilisateur s'étendant entre des première et deuxième parties d'extrémité et comprenant une source lumineuse émettant sur une partie interne du bandeau et des première et deuxième unités d'oreilles configurées pour être portées sur les oreilles d'un utilisateur, les parties d'extrémités et du bandeau sont reliées respectivement aux première et deuxième unités d'oreille de telle sorte qu'en configuration montée le bandeau exerce une compression des unités d'oreille contre la tête de l'utilisateur pour assurer le maintien du dispositif en mains libres, dans lequel les parties d'extrémité portent chacune des moyens d'accrochage libérable du bandeau configurées pour coopérer respectivement avec des moyens d'accrochage complémentaires portés par les unités d'oreille du type à encliquetage, une partie d'extrémité du bandeau comprenant une patte flexible pourvue d'une boucle, et dans lequel les moyens d'accrochage sont configurés pour autoriser, dans la position d'encliquetage, une rotation relative du bandeau par rapport aux unités d'oreilles.

Grâce à l'invention, le dispositif permet un traitement de toute la surface de la tête sans être plus encombrant, par exemple, qu'un casque d'écouteurs audio classique. En effet, grâce à la possibilité de faire varier la position angulaire du bandeau, bien que la bandeau ne couvre qu'une portion de la tête de l'utilisateur, il est possible de couvrir successivement les zones occipitale, pariétale et frontale du cuir chevelu par une simple rotation relative du bandeau par rapport aux unités d'oreilles. Enfin, comme le bandeau lumineux de photothérapie est raccordé par encliquetage, il peut être échangé par n'importe quel accessoire pourvu de moyens d'accrochage identiques.

Un dispositif selon l'invention peut en outre comprendre l'une ou plusieurs des caractéristiques suivantes.

Dans un mode de réalisation préféré de l'invention, l'unité d'oreille comprend une coque externe délimitant des parois dorsale et ventrale et une paroi annulaire périphérique, la coque délimitant une fente de réception sur sa paroi annulaire périphérique pour l'introduction d'une des parties d'extrémités du bandeau dans une gorge d'encliquetage pourvue d'une forme d'encliquetage configurée pour retenir ladite partie d'extrémité.

Dans un mode de réalisation préféré de l'invention, la forme d'encliquetage et la partie d'extrémité sont conformées pour coopérer en rotation l'une par rapport l'autre dans une position d'encliquetage.

Dans un mode de réalisation préféré de l'invention, la coque comprend une partie évidée intérieure et une jupe flexible interne logée à l'intérieur de la partie évidée délimitant la gorge d'encliquetage, la jupe présentant une protubérance externe faisant saillie hors de la coque au travers d'une ouverture formée dans la paroi dorsale de la coque, la protubérance formant un bouton-poussoir configuré pour qu'une pression manuelle sur le bouton provoque une flexion de la jupe propre à assurer le désengagement de la partie d'extrémité et de la forme d'encliquetage permettant la libération de la partie d'extrémité.

Dans un mode de réalisation préféré de l'invention, la jupe présente une paroi dorsale portant la protubérance et une paroi ventrale portant la forme d'encliquetage et deux cloisons latérales reliant en flexion les parois ventrale et dorsale entre elles.

Dans un mode de réalisation préféré de l'invention, les cloisons latérales sont configurées pour limiter le débattement angulaire de la partie d'extrémité.

Dans un mode de réalisation préféré de l'invention, la forme d'encliquetage a la forme d'une bosse sensiblement arrondie.

Dans un mode de réalisation préféré de l'invention, la forme d'encliquetage est pourvue d'une surface crantée ou striée pour générer une indication tactile lors de la rotation du bandeau par rapport aux unités d'oreilles.

Dans un mode de réalisation préféré de l'invention, une partie d'extrémité du bandeau comprend une patte flexible pourvue d'une boucle.

Dans un mode de réalisation préféré de l'invention, le bandeau comprend un boîtier pourvu d'un port connecteur agencé au travers d'une ouverture du boîtier pour réaliser une interface à couplage magnétique encliquetable avec une prise connecteur complémentaire pour l'alimentation électrique de la source lumineuse.

Dans un mode de réalisation préféré de l'invention, l'unité d'oreille comprend une unité de haut-parleur propre à émettre un signal audio.

Dans un mode de réalisation préféré de l'invention, le dispositif comprend des moyens de pilotage, tel qu'un microcontrôleur avec un programme chargé en mémoire, de la source lumineuse selon une pluralité de séquences d'allumage.

Ensemble comprenant un dispositif selon l'invention et comprenant en outre au moins une autre unité de tête, **caractérisé en ce que** l'autre unité de tête comprend des moyens d'accrochage libérable identiques aux moyens d'accrochage libérable du bandeau de façon à permettre l'accrochage de cette autre unité de tête directement sur les unités d'oreilles.

Dans un mode de réalisation préféré de l'invention, l'autre unité de tête porte des moyens configurés pour réaliser au moins en partie une fonction choisie parmi une fonction d'éclairage, une fonction décorative, une fonction de traitement de photothérapie du visage, une fonction d'acquisition d'images vidéo

D'autres caractéristiques et avantages de la présente invention ressortiront clairement de la description détaillée donnée ci-après d'un mode de réalisation de l'invention, donne à titre d'exemple non limitatif, en référence aux dessins annexes dans lesquels :
[Fig 1] est une vue en perspective d'un ensemble de dispositif de photothérapie et de son raccordement d'alimentation électrique ;
[Fig 2] est une vue en perspective éclatée du dispositif de la figure 1 ;
[Fig 3] est une vue schématique en coupe du dispositif de photothérapie lorsque le bandeau et l'unité d'oreille sont en configuration séparée ;
[Fig 4] est une vue schématique en coupe du dispositif de photothérapie lorsque le bandeau et l'unité d'oreille sont en configuration encliquetée ;
[Fig 5] est une vue schématique illustrant le dispositif porté sur la tête d'un utilisateur dans une première position dite occipitale, une deuxième position dite pariétale, une troisième position dite frontale.
[Fig 6] est une vue schématique illustrant le dispositif de photothérapie comprenant une autre unité de tête.

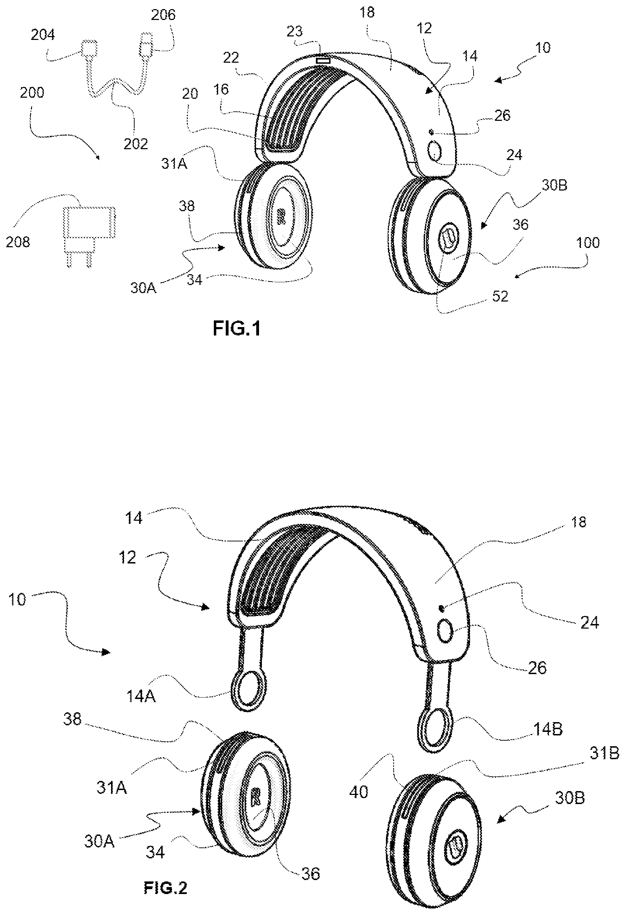

On a représenté de façon schématique sur **la** **figure 1** un ensemble 100 comprenant un dispositif de photothérapie 10 selon l'invention et des moyens 200 de raccordement électrique du dispositif 10 à une source d'alimentation électrique externe (non représentée), telle que par exemple l'alimentation du secteur.

Selon l'invention, le dispositif portatif main libres 10 de traitement par photothérapie comprend une unité principale de tête 12 comportant un bandeau 14 en forme d'arceau équipé d'une source lumineuse 16 et configurée pour être positionné au-dessus de la tête d'un utilisateur.

La source lumineuse 16 comprend de préférence une pluralité de diodes électroluminescentes émettant dans la lumière rouge, par exemple à l'intérieur d'une plage de longueurs d'onde comprises entre 600 nm et 700 nm, et de préférence à la longueur d'onde 630 nm.

Dans l'exemple décrit, le bandeau 14 comprend un boîtier 18 conformé en arceau. Ainsi, le boîtier 18 comprend deux parties interne 20 et externe 22 définies par rapport à la courbure générale du bandeau 14. Ces parties interne 20 et externe 22 délimitent entre elles un logement, par exemple pour la source lumineuse 16 et une plaque de circuit imprimé formant support pour cette source 16. Une telle plaque de circuit imprimé peut être réalisée dans un matériau flexible pour épouser la forme arquée du logement intérieur du boîtier 18. De préférence, la partie interne 20 comprend une fenêtre d'émission 22 de la lumière émise par la source lumineuse 16 réalisée dans un matériau diffusant, par exemple une matière plastique telle que du polycarbonate.

Par ailleurs, comme cela est illustré sur **la** **figure 1****,** le boîtier 18 est, dans cet exemple, pourvu d'un port connecteur 23 agencé au travers d'une ouverture du boîtier 18 pour réaliser par exemple une interface d'alimentation électrique avec les moyens de raccordement 200. Par exemple, cette interface peut être du type à couplage magnétique encliquetable avec une prise connecteur complémentaire pour l'alimentation électrique de la source lumineuse 14.

De préférence, les moyens de raccordement électrique 200 permettent de recharger électriquement une unité interne de batterie logée dans le boîtier 18 du dispositif 10. A cet effet, une ouverture est pratiquée sur un bord périphérique du boîtier 18 et, dans ce mode de réalisation et comme cela est illustré sur les figures, le port connecteur 23 permet de former une interface connectable accessible depuis l'extérieur du boîtier 18 pour la connexion d'une prise connecteur 208 des moyens de raccordement 200, complémentaire au port connecteur 23. De préférence, cette interface connectable est du type à couplage magnétique et encliquetage.

A cet effet, de préférence, les moyens de raccordement 200 comprennent une prise connecteur séparable 204 pour le raccordement à l'alimentation électrique du secteur par exemple. La prise 204 et le port 23 peuvent présenter une symétrie de rotation de telle sorte qu'une rotation à 180 degrés de l'un ou l'autre de la prise ou du port, dans le sens horaire ou antihoraire, entraîne une connexion électrique identique, éliminant ainsi la nécessité de vérifier l'alignement lors de l'établissement d'une connexion. En variante, la prise 204 et le port 23 ne peuvent être raccordés que dans une unique position. Dans ce cas, les éléments aimantés de l'interface à couplage magnétique sont polarisés de telle manière que l'utilisateur ne peut connecter l'interface que dans une unique position. Dans cette variante, l'interface est ainsi munie d'un détrompeur de sorte qu'en cas d'inversion du sens relatif de la prise 204 et du port 23, les éléments aimantés se repoussent.

Par ailleurs, la prise connecteur 204 est reliée également à une première extrémité d'un câble d'alimentation électrique 202, ce câble 202 étant pourvu à son autre extrémité d'un connecteur 206 de type « USB » ou équivalent. Ce connecteur 206 est de préférence apte à être engagé dans une prise mâle 208 de format standard pour le raccordement électrique à un réseau d'alimentation électrique domestique.

Dans l'exemple décrit, le boîtier 18 comprend encore un bouton marche/arrêt 24 et un voyant lumineux 26 d'indication de fonctionnement et loge par exemple d'autres composants électroniques non décrits.

En outre, le dispositif 10 comprend des première 30A et deuxième 30B unités d'oreilles configurées pour être portées sur les oreilles de l'utilisateur. En particulier, le bandeau 14 comprend deux parties d'extrémités 14A, 14B accrochées respectivement aux première 30A et deuxième 30B unités d'oreille de telle sorte qu'en configuration montée le bandeau 14 exerce une compression des unités d'oreille 30A, 30B contre la tête de l'utilisateur pour assurer le maintien du dispositif à mains libres 10 sur la tête de l'utilisateur. Par exemple, le bandeau 14 est élastiquement déformable pour venir s'adapter à la forme de la tête de l'utilisateur par retour élastique.

Conformément à l'invention, les extrémités 14A, 14B portent chacune des moyens d'accrochage libérable du type à encliquetage du bandeau 14 configurés pour coopérer respectivement avec des moyens d'accrochage complémentaires 31A, 31B portés respectivement par les unités d'oreille 30A, 30B. En particulier, les moyens d'accrochage 14A, 14B, 31A, 31B sont configurés pour autoriser, dans la position d'encliquetage, une rotation relative du bandeau 14 par rapport aux unités d'oreilles 30A, 30B vers les oreilles de l'utilisateur.

De préférence, comme cela est illustré sur **la** **figure 2****,** l'unité d'oreille 30A ou 30B comprend une coque externe 32 délimitant des parois dorsale 34 et ventrale 36 et une paroi annulaire périphérique 38. Par exemple, la coque 32 délimite une fente de réception 40 sur sa paroi annulaire périphérique pour l'introduction de la partie d'extrémité 14A du bandeau 14.

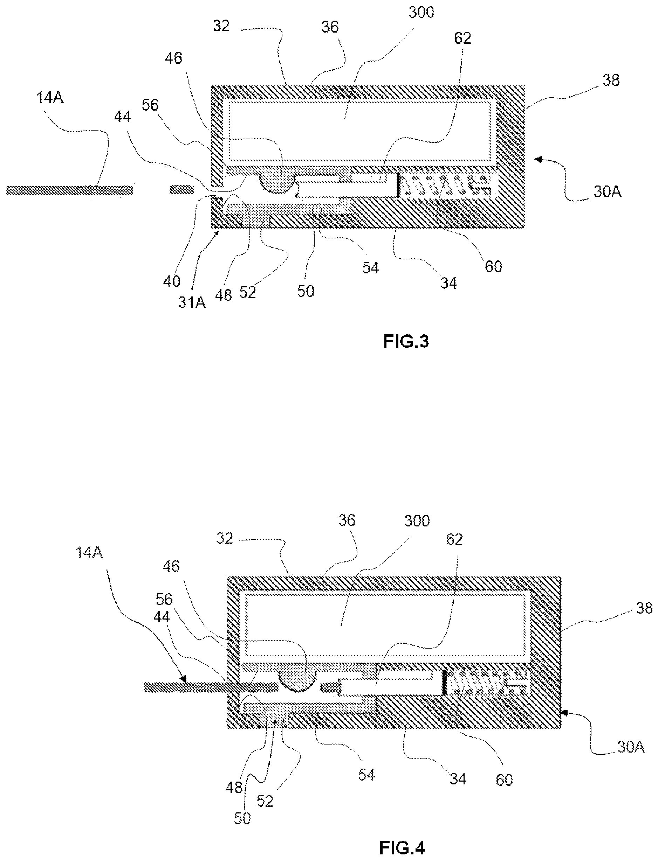

En référence **aux** **figures 3 et 4****,** la suite de la description ne décrira qu'une unité d'oreille 30A, en l'occurrence l'unité d'oreille gauche 30A, étant entendu que le dispositif 10 comporte deux unités d'oreilles 30A, 30B identiques montées symétriquement de part et d'autre du bandeau 14.

**Les** **figures 3 et 4** illustrent le mécanisme des moyens d'accrochage par encliquetage selon un mode de réalisation préféré de l'invention. Bien entendu, ces moyens d'accrochage peuvent présenter d'autres configurations sans sortir pour autant du cadre de l'invention défini par les revendications.

**Sur la** **figure 3****,** on retrouve la coque 32 de l'unité d'oreille 30A pourvue de sa fente d'encliquetage 40 ménagée sur sa paroi annulaire périphérique 38 permettant l'introduction de la boucle de la partie d'extrémité 14A du bandeau 14. Dans cet exemple, la boucle de la partie d'extrémité 14A est insérée à l'intérieur d'une gorge d'encliquetage 44 ménagée à l'intérieur de la coque 32.

De préférence, la gorge d'encliquetage 44 est pourvue d'une forme d'encliquetage 46 configurée pour retenir la partie d'extrémité 14A du bandeau 14 tout en autorisant une rotation relative du bandeau 14 par rapport aux unités d'oreilles 30A, 30B en configuration montée sur les oreilles de l'utilisateur.

En outre, de préférence, comme cela est illustré sur ces figures, la coque 32 de l'unité d'oreille 30A comprend une partie évidée intérieure 48 ou alésage interne 48 et une jupe flexible interne 50 logée à l'intérieur de l'alésage interne 48 délimitant la gorge d'encliquetage 44. De préférence, la jupe 50 présente une protubérance externe 52 faisant saillie hors de la coque 32 au travers d'une ouverture formée dans la paroi dorsale 34 de la coque 32. Par exemple, la protubérance 52 forme un bouton poussoir configuré pour qu'une pression manuelle sur le bouton provoque une flexion de la jupe 50 propre à assurer le désengagement de la partie d'extrémité 14A et de la forme d'encliquetage 46 permettant la libération de la partie d'extrémité 14A préalablement engagée.

Dans l'exemple décrit, la jupe 50 présente une paroi dorsale 54 portant sur une face externe la protubérance 52 et une paroi ventrale 56 portant sur une face interne la forme d'encliquetage 46 et deux cloisons latérales (non visibles sur les figures) reliant en flexion les parois ventrale 54 et dorsale 56, entre elles. Dans l'exemple décrit, lorsqu'une pression manuelle est exercée sur la protubérance, la paroi dorsale 54 se déplace et la paroi ventrale 56 est également entraînée en flexion par les deux cloisons latérales reliées à la paroi dorsale 54. Bien entendu, d'autres formes d'enclenchement et de libération de l'encliquetage peuvent être envisagés sans sortir pour autant du cadre de l'invention.

Dans l'exemple illustré sur les figures, la coque 32 comprend également un alésage cylindrique ou logement cylindrique pour retenir un élément élastique 60, par exemple un ressort fonctionnant en compression. Par ailleurs, de façon optionnelle, la coque 32 comprend encore une clavette d'éjection 62 reliée mécaniquement à l'élément élastique 60.

Dans cet exemple, en position de repos, la clavette 62 est entraînée, par le ressort déployé 60, dans la gorge d'encliquetage 44 tandis que l'introduction de la boucle de la partie d'extrémité 14A provoque le refoulement de la clavette d'éjection 62 jusqu'à la mise en place de la forme d'encliquetage 46 à l'intérieur de la boucle de la partie d'extrémité 14A.

Dans le mode de réalisation préféré de l'invention, une partie d'extrémité 14A, 14B du bandeau 14 comprend une patte flexible pourvue d'une boucle.

De préférence, la forme d'encliquetage 46 a la forme d'une bosse sensiblement arrondie et la partie d'extrémité 14A comprend une boucle. Par ailleurs, dans l'exemple décrit, la bosse 46 est pourvue d'un relief en surface configuré pour former des crans générant ainsi une indication tactile lors de la rotation passage de crans. Par exemple, la bosse 46 est striée ou crantée sur sa périphérie.

Par ailleurs, dans le mode de réalisation décrit, les cloisons latérales de la jupe 50 sont configurées pour limiter le débattement angulaire en rotation de la partie d'extrémité 14A à l'intérieur de la fente 40.

Dans l'exemple illustré, par ailleurs, de façon tout à fait optionnelle, l'unité d'oreille 30A peut comprendre une unité de haut-parleur propre à émettre un signal audio. Cette unité de haut-parleur est représentée schématiquement par un rectangle référencée 300 sur **les** **figures 3** et **4** mais ne sera pas davantage détaillé.

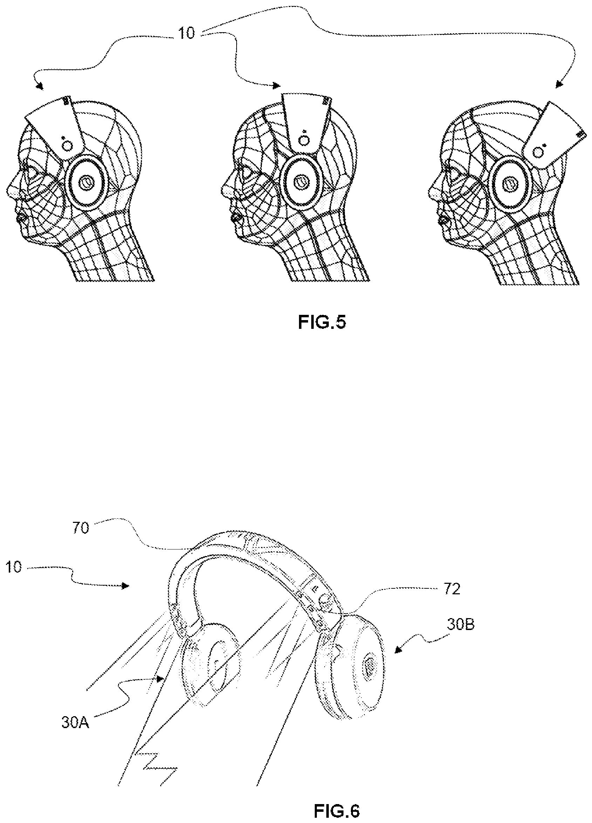

Selon l'invention, l'ensemble 100 comprend en outre au moins une autre unité de tête illustrée sur **la** **figure 6****.** Cette autre unité de tête 70 comprend des moyens d'accrochage libérable identiques aux moyens d'accrochage libérable du bandeau 14 de façon à permettre l'accrochage de cette autre unité de tête 70 directement sur les unités d'oreilles 30A, 30B.

De préférence, l'autre unité de tête 70 porte des moyens configurés pour réaliser au moins en partie une fonction choisie parmi une fonction d'éclairage, une fonction décorative, une fonction de traitement de photothérapie du visage, une fonction d'acquisition d'images vidéo, par exemple une caméra sportive de type GOPRO^{®}. Dans l'exemple illustré sur **la** **figure 6****,** l'autre unité de tête 70 comprend une fonction lumineuse réalisée par exemple par une source lumineuse 72 de lumière blanche.

On va maintenant décrire les principaux aspects du dispositif de photothérapie portatif selon l'invention en référence notamment à **la** **figure 5****.**

Initialement, l'utilisateur souhaite traiter le cuir chevelu. Il sélectionne l'unité de tête 12 pour le traitement par photothérapie. Il accroche les parties d'extrémités 14A, 14B aux unités d'oreille 30A, 30B. Puis, il positionne le dispositif 10 au-dessus de sa tête de telle sorte que le bandeau 14 s'étende au-dessus de la zone du cuir chevelu à traiter et les unités d'oreilles 30A, 30B autour des oreilles. Lors de cette étape, l'utilisateur écarte légèrement les unités d'oreilles 30A, 30B l'une de l'autre par déformation élastique du bandeau 14 puis relâche le bandeau 14, ce dernier venant par retour élastique comprimer les unités d'oreilles 30A, 30B contre les oreilles de l'utilisateur.

Puis, l'utilisateur enclenche le programme de photothérapie en appuyant sur le bouton marche/arrêt 26. Le voyant lumineux 24 indique alors par une lumière que le programme de photothérapie est activé. La source lumineuse 16 éclaire le cuir chevelu pendant une durée de traitement prédéfinie pouvant être programmée. Une fois ce programme terminé, l'utilisateur fait pivoter le bandeau 14 tout en maintenant les unités d'oreilles à leur position initiale. Le bandeau 14 est alors déplacé par rotation d'une zone arrière vers une zone avant de la tête de l'utilisateur afin de traiter toute la surface de la tête.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Dispositif de photothérapie portatif (10) mains libres, du type comprenant une unité principale (12) de tête comportant un bandeau (14) en forme d'arceau configuré pour être positionné au-dessus de la tête d'un utilisateur s'étendant entre des première (14A) et deuxième (14B) parties d'extrémité et comprenant une source lumineuse (16) émettant sur une partie interne (20) du bandeau (14) et des première (30A) et deuxième (30B) unités d'oreilles configurées pour être portées sur les oreilles d'un utilisateur, les parties d'extrémités (14A) et (14B) du bandeau (14) sont reliées respectivement aux première (30A) et deuxième (30B) unités d'oreille de telle sorte qu'en configuration montée le bandeau (14) exerce une compression des unités d'oreille (30A, 30B) contre la tête de l'utilisateur pour assurer le maintien du dispositif (10) en mains libres, dans lequel les parties d'extrémité (14A, 14B) portent chacune des moyens d'accrochage libérable du bandeau (14) configurées pour coopérer respectivement avec des moyens d'accrochage complémentaires (31A, 31B) portés par les unités d'oreille (30A, 30B) du type à encliquetage, une partie d'extrémité (14A, 14B) du bandeau (14) comprenant une patte flexible pourvue d'une boucle et dans lequel les moyens d'accrochage (14A, 31A ; 14B, 31B) sont configurés pour autoriser, dans la position d'encliquetage, une rotation relative du bandeau (14) par rapport aux unités d'oreilles (30A, 30B).

2. Dispositif (10) selon la revendication précédente, dans lequel l'unité d'oreille (30A, 30B) comprend une coque externe (32) délimitant des parois dorsale (34) et ventrale (36) et une paroi annulaire périphérique (38), la coque (32) délimitant une fente de réception (40) sur sa paroi annulaire périphérique (38) pour l'introduction d'une des parties d'extrémités (14A, 14B) du bandeau (14) dans une gorge d'encliquetage (44) pourvue d'une forme d'encliquetage (46) configurée pour retenir la dite partie d'extrémité (14A, 14B).

3. Dispositif (10) selon la revendication précédente, dans lequel la forme d'encliquetage (46) et la partie d'extrémité (14A, 14B) sont conformées pour coopérer en rotation l'une par rapport l'autre dans une position d'encliquetage.

4. Dispositif (10) selon la revendication précédente, dans lequel la coque (32) comprend une partie évidée intérieure (48) et une jupe flexible interne (50) logée à l'intérieur de la partie évidée (48) délimitant la gorge d'encliquetage (44), la jupe (50) présentant une protubérance externe (52) faisant saillie hors de la coque (32) au travers d'une ouverture formée dans la paroi dorsale (34) de la coque (32), la protubérance (52) formant un bouton-poussoir configuré pour qu'une pression manuelle sur le bouton (52) provoque une flexion de la jupe (50) propre à assurer le désengagement de la partie d'extrémité (14A, 14B) et de la forme d'encliquetage (46) permettant la libération de la partie d'extrémité (14A, 14B).

5. Dispositif (10) selon la revendication précédente, dans lequel la jupe (50) présente une paroi dorsale (54) portant la protubérance (52) et une paroi ventrale (56) portant la forme d'encliquetage (46) et deux cloisons latérales reliant en flexion les parois ventrale (52) et dorsale (54) entre elles.

6. Dispositif (10) selon la revendication précédente, dans lequel les cloisons latérales sont configurées pour limiter le débattement angulaire de la partie d'extrémité (14A, 14B).

7. Dispositif (10) selon l'une quelconque des revendications 2 à 6, dans lequel la forme d'encliquetage (46) a la forme d'une bosse sensiblement arrondie.

8. Dispositif (10) selon la revendication précédente, dans lequel la forme d'encliquetage (46) est pourvue d'une surface crantée ou striée pour générer une indication tactile lors de la rotation du bandeau (14) par rapport aux unités d'oreilles (30A, 30B).

9. Dispositif (10) selon l'une quelconque des revendications précédentes, dans lequel le bandeau (14) comprend un boîtier (18) pourvu d'un port connecteur (23) agencé au travers d'une ouverture du boîtier (18) pour réaliser une interface à couplage magnétique encliquetable avec une prise connecteur complémentaire pour l'alimentation électrique de la source lumineuse (16).

10. Dispositif (10) selon l'une quelconque des revendications précédentes, dans lequel l'unité d'oreille (30A, 30B) comprend une unité de haut-parleur propre à émettre un signal audio.

11. Dispositif (10) selon l'une quelconque des revendications précédentes, comprenant des moyens de pilotage, tel qu'un microcontrôleur avec un programme chargé en mémoire, de la source lumineuse (16) selon une pluralité de séquences d'allumage.

12. Ensemble (100) comprenant un dispositif (10) selon l'une quelconque des revendications précédentes et comprenant en outre au moins une autre unité de tête (70), **caractérisé en ce que** l'autre unité de tête (70) comprend des moyens d'accrochage libérable identiques aux moyens d'accrochage libérable du bandeau (14) de façon à permettre l'accrochage de cette autre unité de tête directement sur les unités d'oreilles (30A, 30B).

13. Ensemble (100) selon la revendication précédente, dans lequel l'autre unité de tête (70) porte des moyens configurés pour réaliser au moins en partie une fonction choisie parmi une fonction d'éclairage, une fonction décorative, une fonction de traitement de photothérapie du visage, une fonction d'acquisition d'images vidéo.

## Patentansprüche

1. Tragbare, freihändige Phototherapievorrichtung (10) des Typs, der eine Hauptkopfeinheit (12) mit einem Stirnband umfasst. (14) in Form eines Bügels, der so konfiguriert ist, dass er über dem Kopf eines Benutzers positioniert werden kann, der sich zwischen einem ersten (14A) und einem zweiten (14B) Endabschnitt erstreckt und eine Lichtquelle (16), die auf einen inneren Abschnitt (20) des Stirnbands (14) strahlt, und eine erste (30A) und eine zweite (30B) Ohreinheit umfasst, die so konfiguriert sind, dass sie an den Ohren eines Benutzers getragen werden können, die Endabschnitte (14A) und (14B) des Kopfbands (14) jeweils mit der ersten (30A) und der zweiten (30B) Ohreinheit verbunden sind, so dass das Kopfband (14) in der montierten Konfiguration eine Kompression der Ohreinheiten (30A, 30B) gegen den Kopf des Benutzers ausübt, um das freihändige Halten der Vorrichtung (10) zu gewährleisten, wobei die Endabschnitte (14A, 14B) jeweils lösbare Einhakmittel des Kopfbandes (14) tragen, die so konfiguriert sind, dass sie jeweils mit komplementären Einhakmitteln (31A, 31B) zusammenwirken, wobei die komplementären Einhakmittel (31A, 31B) jeweils mit den Endabschnitten 14A, 14B) des Kopfbandes (14) zusammenwirken, 31B), die von den Ohreinheiten (30A, 30B) vom Einschnapptyp getragen werden, wobei ein Endabschnitt (14A, 14B) des Kopfbandes (14) eine flexible Lasche umfasst, die mit einer Schnalle versehen ist, und wobei die Einhakmittel (14A, 31A ; 14B, 31B) so konfiguriert sind, dass sie in der Einrastposition eine relative Drehung des Kopfbands (14) in Bezug auf die Ohreneinheiten (30A, 30B) zulassen.

2. Vorrichtung (10) nach dem vorhergehenden Anspruch, wobei die Ohreinheit (30A, 30B) eine äußere Schale (32), die eine Rückenwand (34) und eine Bauchwand (36) begrenzt, und eine ringförmige Umfangswand (38) umfasst, die Schale (32) an ihrer ringförmigen Umfangswand (38) einen Aufnahmeschlitz (40) zum Einführen eines der Endabschnitte (14A, 14B) des Kopfbandes (14) in eine Rastnut (44) begrenzt, die mit einer Rastform (46) versehen ist, die so konfiguriert ist, dass sie den Endabschnitt (14A, 14B) festhält.

3. Vorrichtung (10) nach dem vorhergehenden Anspruch, wobei die Einrastform (46) und der Endabschnitt (14A, 14B) so geformt sind, dass sie in einer Einrastposition drehbar relativ zueinander zusammenwirken.

4. Vorrichtung (10) nach dem vorhergehenden Anspruch, wobei die Schale (32) einen inneren ausgesparten Abschnitt (48) und eine innere flexible Schürze (50) aufweist, die in dem ausgesparten Abschnitt (48) untergebracht ist, der die Rastnut (44) begrenzt, wobei die Schürze (50) einen äußeren Vorsprung (52) aufweist, der aus der Schale (32) durch eine äußere Ausbuchtung (52) vorsteht, die durch die Rastnut (44) hindurch verläuft. Öffnung, die in der Rückenwand (34) des Rumpfes (32) ausgebildet ist, wobei der Vorsprung (52) einen Druckknopf bildet, der so konfiguriert ist, dass ein manueller Druck auf den Knopf (52) eine Biegung der Schürze bewirkt. (50), die geeignet ist, das Lösen des Endabschnitts (14A, 14B) zu gewährleisten, und der Einrastform (46), die die Freigabe des Endabschnitts (14A, 14B) ermöglicht.

5. Vorrichtung (10) nach dem vorhergehenden Anspruch, wobei die Schürze (50) eine Rückenwand 54), die den Vorsprung (52) trägt, und eine Bauchwand (56), die die Rastform (46) trägt, sowie zwei Seitenwände aufweist, die die Bauchwand (52) und die Rückenwand biegsam miteinander verbinden (54) untereinander.

6. Vorrichtung (10) nach dem vorhergehenden Anspruch, wobei die Seitenwände so gestaltet sind, dass sie die Winkelauslenkung des Endabschnitts (14A, 14B) begrenzen.

7. Vorrichtung (10) nach einem der Ansprüche 2 bis 6, wobei die Einrastform (46) die Form eines im Wesentlichen abgerundeten Höckers hat.

8. Vorrichtung (10) nach dem vorhergehenden Anspruch, wobei die Einrastform (46) mit einer gezahnten oder geriffelten Oberfläche versehen ist, um eine taktile Anzeige zu erzeugen, wenn das Kopfband (14) in Bezug auf die Ohreneinheiten (30A, 30B) gedreht wird.

9. Vorrichtung (10) nach einem der vorhergehenden Ansprüche, wobei das Stirnband (14) ein Gehäuse (18) umfasst, das mit einem Steckeranschluss (23) versehen ist, der durch eine Öffnung des Gehäuses (18) hindurch angeordnet ist, um eine magnetisch gekoppelte Schnittstelle zu bilden, die mit einem komplementären Steckeranschluss für die Stromversorgung der Lichtquelle (16) einschnappbar ist.

10. Vorrichtung (10) nach einem der vorhergehenden Ansprüche, wobei die Ohreinheit (30A, 30B) eine Lautsprechereinheit umfasst, die zum Ausgeben eines Audiosignals geeignet ist.

11. Vorrichtung (10) nach einem der vorhergehenden Ansprüche, die Mittel zur Steuerung, wie einen Mikrocontroller mit einem in den Speicher geladenen Programm, der Lichtquelle (16) gemäß einer Vielzahl von Zündsequenzen umfasst.

12. Anordnung (100), die eine Vorrichtung (10) nach einem der vorhergehenden Ansprüche umfasst und außerdem mindestens eine weitere Kopfeinheit (70) umfasst, **dadurch gekennzeichnet, dass** die weitere Kopfeinheit (70) lösbare Hakenmittel umfasst, die identisch mit den lösbaren Hakenmitteln des Kopfbandes (14) sind, um so das Aufhängen dieser anderen Kopfeinheit direkt an den Ohreneinheiten (30A, 30B) ermöglichen.

13. Anordnung (100) nach dem vorhergehenden Anspruch, bei der die andere Kopfeinheit (70) Mittel trägt, die so konfiguriert sind, dass sie zumindest teilweise eine Funktion ausführen, die aus einer Beleuchtungsfunktion, einer dekorativen Funktion, einer Funktion zur Phototherapiebehandlung des Gesichts und einer Funktion zur Erfassung von Videobildern ausgewählt ist.

## Claims

1. Hands-free portable phototherapy device (10) of the type comprising a main head unit (12) with a headband. (14) configured to be positioned over a user's head extending between first (14A) and second (14B) end portions and comprising a light source (16) emitting onto an inner portion (20) of the headband (14) and first (30A) and second (30B) ear units configured to be worn over a user's ears, the end portions (14A) and (14B) of the headband (14) are connected to the first (30A) and second (30B) ear units respectively, so that in the fitted configuration the headband (14) presses the ear units (30A, 30B) against the user's head to keep the device (10) hands-free, wherein the end portions (14A, 14B) each carry releasable hooking means of the headband (14) configured to cooperate respectively with complementary hooking (31A, 31B) carried by the snap-type ear units (30A, 30B), one end portion (14A, 14B) of the headband (14) comprising a flexible tab provided with a loop, and wherein the hooking means (14A, 31A ; 14B, 31B) are configured to allow, in the snap-in position, relative rotation of the headband (14) with respect to the ear units (30A, 30B).

2. Device (10) according to the preceding claim, in which the ear unit (30A, 30B) comprises an outer shell (32) delimiting dorsal (34) and ventral (36) walls and an annular peripheral wall (38), the shell (32) delimiting a receiving slot (40) on its peripheral annular wall (38) for insertion of one of the end portions (14A, 14B) of the band (14) into a snap groove (44) provided with a snap form (46) configured to retain said end portion (14A, 14B).

3. Device (10) according to the preceding claim, in which the snap form (46) and the end portion (14A, 14B) are shaped to cooperate rotatably relative to one another in a snap position.

4. Device (10) according to the preceding claim, in which the shell (32) comprises an internal recessed portion (48) and an internal flexible skirt (50) housed inside the recessed portion (48) defining the snap-in groove (44), the skirt (50) having an external protuberance (52) projecting out of the shell (32) through a opening formed in the back wall (34) of the shell (32), the protuberance (52) forming a push-button configured so that manual pressure on the button (52) causes the skirt to flex (50) to ensure disengagement of the end part (14A, 14B) and the snap form (46) to release the end part (14A, 14B).

5. Device (10) according to the preceding claim, in which the skirt (50) has a dorsal wall (54) carrying the protuberance (52) and a ventral wall (56) carrying the snap-in form (46) and two lateral partitions flexibly connecting the ventral (52) and dorsal walls. (54) between them.

6. Device (10) according to the preceding claim, wherein the side partitions are configured to limit the angular deflection of the end portion (14A, 14B).

7. Device (10) according to any one of claims 2 to 6, wherein the snap form (46) has the shape of a substantially rounded hump.

8. Device (10) according to the preceding claim, wherein the snap form (46) is provided with a serrated or ridged surface for generating a tactile indication upon rotation of the headband (14) relative to the ear units (30A, 30B).

9. Device (10) according to any one of the preceding claims, wherein the headband (14) comprises a housing (18) provided a connector port (23) arranged through an opening in the housing (18) to provide a snap-fit magnetically coupled interface with a complementary connector socket for powering the light source (16).

10. Device (10) according to any one of the preceding claims, wherein the ear unit (30A, 30B) comprises a loudspeaker unit suitable for emitting an audio signal.

11. Device (10) according to any of the preceding claims, comprising means for controlling the light source (16) according to a plurality of lighting sequences, such as a microcontroller with a program loaded in memory.

12. Assembly (100) comprising a device (10) according to any of the preceding claims and further comprising at least one further head unit (70), **characterized in that** the further head unit (70) comprises releasable hooking means identical to the releasable hooking means of the headband (14) so as to allow this other head unit to be hooked directly onto the ear units (30A, 30B).

13. Assembly (100) according to the preceding claim, in which the other head unit (70) carries means configured to perform at least in part a function chosen from a lighting function, a decorative function, a facial phototherapy treatment function and a video image acquisition function.
